# EUROPEAN PATENT APPLICATION

(11) **EP 4 708 320 A2**
(43) Date of publication of application: **11.03.2026**
(21) Application number: 26151293.3
(22) Date of filing: 11.12.2020
(51) Int. Cl.: G16H 50/20

(54) **AUTOMATED CHROMATOGRAM ANALYSIS FOR BLOOD TEST EVALUATION**

(30) Priority: 19.12.2019 US 201916721093
(62) Divisional of application: 20902851.3
(71) Applicant: Bio-Rad Laboratories, Inc., Hercules, CA 94547 (US)
(72) Inventor: SCOLARI, Caroline, Hercules, 94547 (US); FRANCISCO, Jaime, Hercule, 94547 (US); GAMBOA, Fe Seneca, Hercules, 94547 (US)
(74) Representative: FRKelly

(57) **Abstract**

A chromatogram analysis tool receives blood test data for a sample and divides the data into regions and determines a best-fit match template for each region. The chromatogram analysis tool determines a best-fit match for each region by comparing the blood test data to a set of templates associated with archetypical shapes of the region. The template with the highest r-squared value for the blood test data is the best-fit template. The chromatogram analysis tool generates a report based on the best-fit match templates for each region, which can indicate medical conditions or recommendations for additional testing.

## Description

### BACKGROUND

### 1. TECHNICAL FIELD

The subject matter described generally relates to analyzing diagnostic testing data, and in particular to computer-aided blood test evaluation.

### 2. BACKGROUND INFORMATION

A hemoglobinopathy is a genetic defect that results in an unusual structure of hemoglobin molecules in an individual's blood. For example, sickle-cell disease is caused by a hemoglobinopathy that can result in the red blood cells forming a rigid sickle shape under certain circumstances. These misshapen red blood cells can obstruct capillaries and restrict blood flow, leading to a range of health problems. In contrast, a thalassemia is a genetic condition that results in reduced hemoglobin production. Some hemoglobinopathies also impact hemoglobin production, and are thus also thalassemias.

Various medical conditions are characterized by the presence of certain hemoglobin variants and the proportions of different variants in the blood. Blood tests provide information about the proportions of different hemoglobin variants in a blood sample. However, interpreting this information can be challenging. Different conditions can have similar impacts on the presence of certain variants. The analysis is further complicated because other environmental and health factors can impact the proportions of the variants present. For example, an unusually large amount of hemoglobin F may indicate a genetic disorder or may indicate that an individual was pregnant or an infant at the time the sample was taken. Furthermore, relatively small amounts of a variant (or change in the amount of a variant present) may be clinically significant, but masked by variants that are present in far larger amounts.

Computer technology provides novel opportunities to analyze blood test data and more reliably distinguish between different response patterns produced by samples containing variants. This can reduce the reliance on human analysts, who may be subject to making errors and require more time and training to reach diagnoses than may be achieved using technology.

### BRIEF DESCRIPTION OF THE DRAWINGS

FIG. 1 is a high-level block diagram illustrating a networked computing environment in which diagnostic data is generated and analyzed, according to one embodiment.
FIG. 2 is a high-level block diagram illustrating a laboratory terminal suitable for use in the networked computing environment of FIG. 1, according to one embodiment.
FIG. 3 is a high-level block diagram illustrating the chromatogram analysis tool of a laboratory terminal, according to one embodiment.
FIG. 4 is a high-level block diagram illustrating an example of a computer suitable for use as a laboratory terminal, according to one embodiment.
FIG. 5 illustrates an example chromatogram, according to one embodiment.
FIG. 6 is a table illustrating an example division of a chromatogram into regions, according to one embodiment.
FIG. 7A shows example visual representations of regions with of chromatogram data overlaid with best-fit match templates.
FIG. 7B shows an example of chromatogram data and a report that might be generated by the chromatogram analysis tool, according to one embodiment.
FIG. 8 shows an example of a report of a plurality of results generated by the chromatogram analysis tool, according to one embodiment.
FIG. 9 is a flow-chart illustrating a method for generating a report for blood chromatography data, according to one embodiment.

### DETAILED DESCRIPTION

The Figures (FIGS.) and the following description describe certain embodiments by way of illustration only. One skilled in the art will readily recognize from the following description that alternative embodiments of the structures and methods may be employed without departing from the principles described. Reference will now be made to several embodiments, examples of which are illustrated in the accompanying figures. It is noted that wherever practicable similar or like reference numbers are used in the figures to indicate similar or like functionality.

### OVERVIEW AND BENEFITS

A chromatogram analysis tool is used as part of a laboratory blood test system to identify genetic conditions based on the relative proportions of various types of hemoglobin in a sample. The blood test system generates chromatography data from the sample. The chromatogram analysis tool identifies regions of the chromatography data and, for each region, determines a match between the chromatography data in that region and one of a set of possible templates. The regions may have a predetermined size. The templates are of archetypical shapes of the hemoglobin data within the corresponding region, and may be constructed, portions of individual or pooled real exemplary chromatograms, or combinations of real and/or constructed exemplary chromatograms.

The chromatogram analysis tool generates a report based on the best-fit matches. The report may indicate one or more possible medical conditions. The report may also include additional comments and notes, such as suggestions for additional testing that should be performed, common diagnosis pitfalls, additional information about the corresponding condition (e.g., demographic factors that correlate with diagnosis), possible reproductive risks, and the like.

The analysis of regions of the chromatogram using templates has several advantages. First, it may help with result interpretation, enabling laboratories to deliver more standardized results without the need for additional training. In fact, it may reduce the amount of training required for laboratory technicians to operate efficiently. Second, it may enable results to be compared substantially in real time with large databases of reference cases that are available on-line, which may result in more accurate preliminary identifications of potential conditions. Third, by applying templates to regions, variations in scaling are inherently incorporated in the templates corresponding to each region. Thus, matching regions can provide greater accuracy over approaches that match templates to the chromatogram as a whole. Fourth, the method does not rely on peaks not found in normal samples to be integrated and assigned to a specific window. Fifth, the report can result in suggestions for next steps in reaching a diagnosis, which can reduce reliance on human-made connections between test results and possible causes. In some cases, the next steps can be triggered automatically or semi-automatically (e.g., if the required data for the next step is already available in a database), reducing the time taken to complete the testing process.

### EXAMPLE SYSTEMS

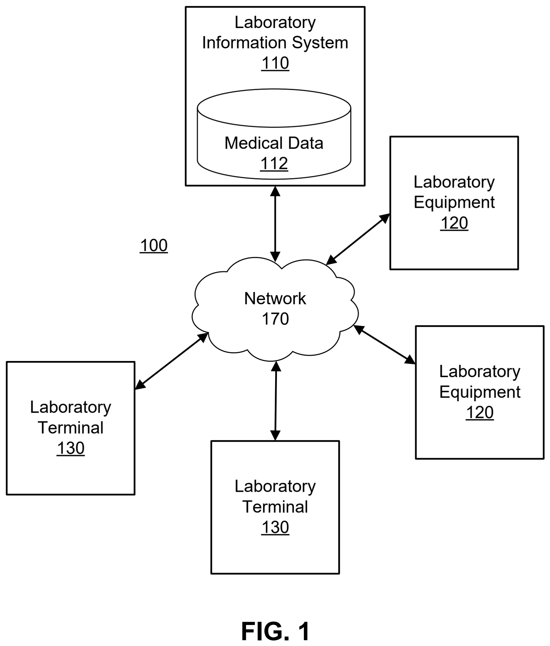

FIG. 1 shows one embodiment of a networked computing environment 100 in which diagnostic data is generated and analyzed. In the embodiment shown in FIG. 1, the networked computing environment includes a laboratory information system (LIS) 110, laboratory equipment 120, and laboratory terminals 130, all connected via a network 170. Although two items of laboratory equipment 120 and two laboratory terminals 130 are shown, a given deployment may include any amount of equipment and any number of terminals (including just a single terminal). In other embodiments, the networked computing environment 100 contains different or additional elements. In addition, the functions may be distributed among the elements in a different manner than described. For example, each item of laboratory equipment 120 may include a computer system that provides the functionality of a laboratory terminal 130.

The LIS 110 is a computer-based system that supports the operations of the laboratory. In various embodiments, the LIS 110 provides tools that help technicians and other users function in the laboratory efficiently. For example, the LIS 110 might provide data tracking, automated backup, data exchange, work flow management, sample management, data analysis, data mining, instrument management, report generation, data auditing, and the like. In the embodiment shown in FIG. 1, the LIS 110 stores medical data 112. The medical data 112 is stored on one or more computer readable media, such as a hard drive. The medical data 112 can include patient records, test results, medical literature, and the like. One of skill in the art will recognize other functionality that the LIS 110 may provide and other types of data that may be stored as part of the medical data 112.

The laboratory equipment 120 is one or more devices that perform medical tests. In one embodiment, the laboratory equipment 120 includes a chromatography system that produces a chromatogram indicating the relative proportions of different variants of hemoglobin present in a sample. An example of such a system is the D-100^{™} produced by Bio-Rad^{™}. The laboratory equipment 120 can also include devices that perform other tests, such as DNA testing, urine testing, and the like. By identifying a possible medical condition, a chromatogram analysis tool may trigger a series of tests for aiding in differential diagnosis of the sample, e.g., a sickling test, a stability test (isopropanol test), electrophoresis tests, MS/MS, molecular studies, and the like.

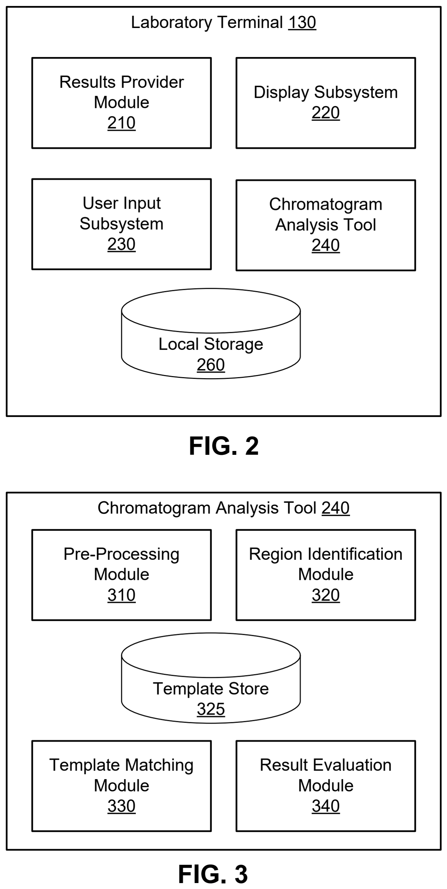

The laboratory terminals 130 are computing devices with which users interact with the LIS 110 and lab equipment 120. In various embodiments, a technician initiates a test on a sample using a terminal 130 that includes a chromatogram analysis tool. The terminal 130 presents a report generated by the chromatogram analysis tool including results analysis and suggestions. In one embodiment, the technician approves the report and it is sent to the LIS 110 for storage. In another embodiment, a laboratory supervisor must also approve the report (e.g., using a second terminal 130). The terminal 130 may also send instructions (e.g., to the LIS 110) to initiate additional tests or provide the results of previously conducted tests based on the recommendations generated by the chromatogram analysis tool. Embodiments of the terminal 130, and in particular operation of the chromatogram analysis tool, are described in additional detail below, with reference to FIGS. 2 and 3.

The network 170 provides the communication channels via which the other elements of the networked computing environment 100 communicate. The network 170 can include any combination of local area or wide area networks, using both wired or wireless communication systems. In one embodiment, the network 170 uses standard communications technologies or protocols. For example, the network 170 can include communication links using technologies such as Ethernet, 802.11, worldwide interoperability for microwave access (WiMAX), 3G, 4G, code division multiple access (CDMA), digital subscriber line (DSL), etc. Examples of networking protocols used for communicating via the network 170 include multiprotocol label switching (MPLS), transmission control protocol/Internet protocol (TCP/IP), hypertext transport protocol (HTTP), simple mail transfer protocol (SMTP), and file transfer protocol (FTP). Data exchanged over the network 170 may be represented using any suitable format, such as hypertext markup language (HTML) or extensible markup language (XML). In one embodiment, some or all of the components are connected using an RS-232 serial connection. In some embodiments, all or some of the communication links of the network 170 may be encrypted using any suitable technique or techniques.

FIG. 2 shows one embodiment of a laboratory terminal 130 suitable for use in the networked computing environment 100 of FIG. 1. In the embodiment shown in FIG. 2, the laboratory terminal 130 includes a results provider 210, a display subsystem 220, a user input subsystem 230, a chromatogram analysis tool 240, and local storage 260. In other embodiments, the laboratory terminal 130 contains different or additional elements. In addition, the functions may be distributed among the elements in a different manner than described.

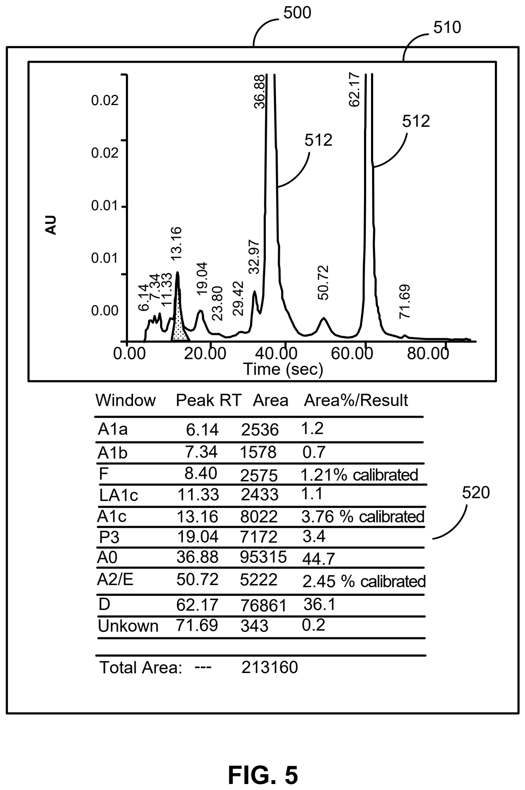

The results provider module 210 interfaces with laboratory equipment 120 to obtain medical data. In one embodiment, the medical data is blood chromatography data that the results provider module 210 uses to create a chromatogram. Alternatively, the chromatogram may be generated by the lab equipment 120 (or elsewhere in the networked computing environment 100) and provided as input to the results provider module 210. FIG. 5 shows an example of a chromatogram 500, according to one embodiment. The chromatogram 500 includes a visual representation of the data 510 and a data table 520. The visual representation 510 includes a plot of detector response over time that includes several peaks 512 (of which only two are labelled for clarity). The data table 520 identifies the retention time (i.e., the time at which the strongest detector response was observed for a peak 512) in various windows expected to correspond to different variants of hemoglobin (e.g., A1a, A1b, F, etc.). The data table 520 also includes the area of each peak 512 (which corresponds to the total amount of the given variant present in the sample) and result reported for each peak.

Referring back to FIG. 2, the display subsystem 220 presents information and controls to a user (e.g., a laboratory scientist). In one embodiment, the display subsystem 220 provides controls with which a technician initiates a test by the laboratory equipment 120. The display subsystem 220 then provides controls to enable the operator to view and analyze the results of the test (e.g., using the chromatogram analysis tool 240). The display subsystem 220 may also provide other functionality, such as viewing patient records, configuring the laboratory equipment 120, viewing status/maintenance data, and the like.

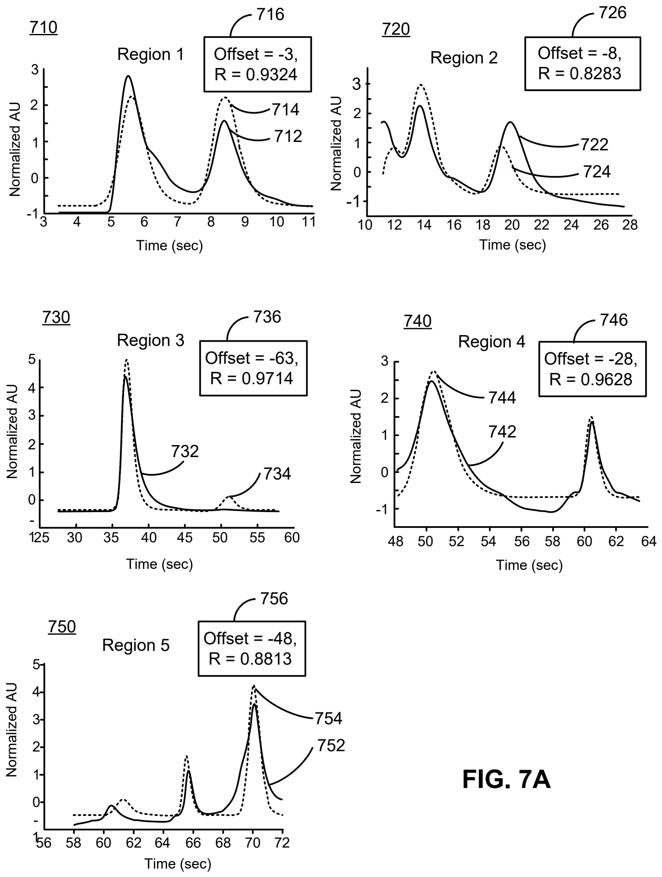

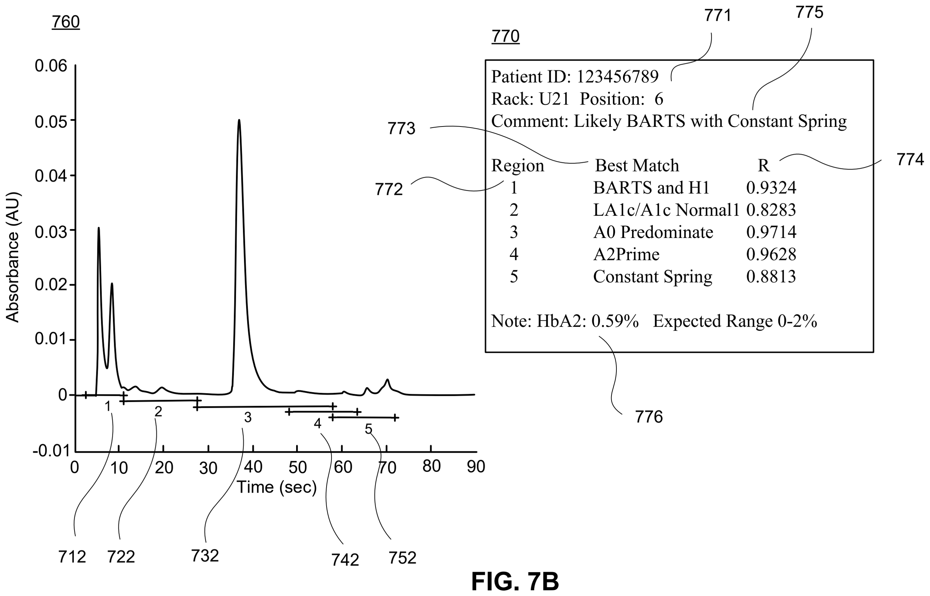

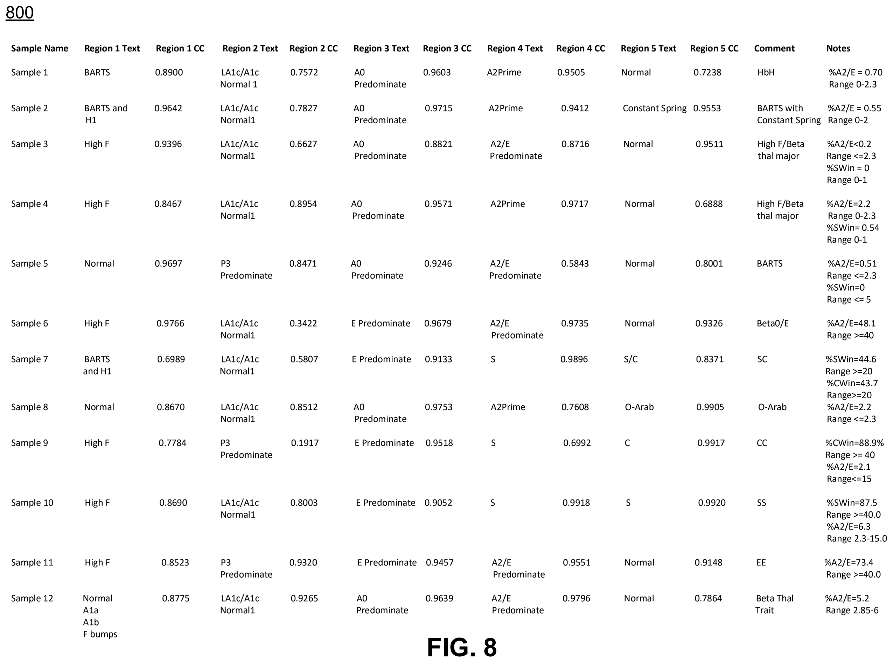

The user input subsystem 230 receives input from a user (e.g., a laboratory scientist or supervisor) and provides it to other elements of the terminal 130. In one embodiment, the user input subsystem 230 includes a touch screen. Controls are presented on the touch screen enabling the user to control the laboratory equipment 120 or interact with the chromatogram analysis tool 240. Further details of embodiments of the user interface provided by the user input subsystem 230 are provided below, with reference to FIGS. 7 and 8.

The chromatogram analysis tool 240 analyzes the data provided by the results provider module 210 to generate a report. In various embodiments, the chromatogram analysis tool 240 subdivides the chromatogram into regions and matches each region to a set of templates corresponding to possible morphologies of the region to find a best-fit match. The chromatogram analysis tool 240 then generates a report based on the best-fit matches for each region and includes comments regarding interpretation of the result. The report may additionally include a likelihood that each best-fit match is correct or a recommendation for further testing that will allow a definitive diagnosis. For example, if the results suggest the subject may be a carrier of an inheritable blood disorder, the chromatogram analysis tool 240 might recommend a DNA test for verification if the subject is considering having children. In one embodiment, the chromatogram analysis tool 240 may automatically trigger further analysis if the required data or equipment is available and update the report accordingly. Details of various embodiments of the chromatogram analysis tool 240 are described in greater detail below, with reference to FIG. 3.

FIG. 3 shows one embodiment of the chromatogram analysis tool 240 of the laboratory terminal 120 shown in FIG. 2. In the embodiment shown in FIG. 3, the chromatogram analysis tool 240 includes a pre-processing module 310, a region identification module 320, a template store 325, a template matching module 330, and a result evaluation module 340. In other embodiments, the chromatogram analysis tool 240 contains different or additional elements. In addition, the functions may be distributed among the elements in a different manner than described.

The pre-processing module 310 performs a variety of baseline operations and quality checks prior to further analysis of the data. In some embodiments, the pre-processing module 310 performs a baseline subtraction on the chromatogram prior to any subsequent analyses. In some embodiments, the pre-processing module 310 may perform initial analyses of the chromatogram. For example, the pre-processing module 310 may calculate heights, areas, and generate calibrated and non-calibrated results. The pre-processing module 310 may also calculate special sums from these calibrated and non-calibrated results that combine data from one or more peaks to aid in efficient analysis.

In some embodiments, the pre-processing module 310 analyzes the quality of the data. In one such embodiment, the quality analysis checks for features in the data that may indicate a high likelihood of inaccurate results. For example, the quality analysis module 310 can compare the total area for a chromatogram to a minimum area threshold and flag the test data as low-quality if the total area is less than the threshold. In this example, the pre-processing module 310 may use the special sums calculated as described above. If the test data is flagged as low-quality data, the pre-processing module 310 may end the analysis and indicate that a new test should be performed. This can prevent time and resources being wasted on further analysis of data that is unreliable. In such cases, the pre-processing module 310 may automatically trigger retesting of the sample. In another example, the pre-processing module 310 might look at the width, exponentially modified Gaussian fit sigma and tau values, or indicators derived from exponentially modified Gaussian fit sigma and tau values of a known peak (e.g., the A1c or A2 peaks) and add a warning comment if thresholds are exceeded. Other examples include the quality analysis module 310 checking for uneven baselines and highly asymmetrical peaks (e.g., peak tailing) using exponentially modified Gaussian tau/sigma ratio or another indicator.

The region identification module 320 divides the chromatogram into regions. The region identification module 320 uses features of the chromatogram and/or absolute or normalized times to determine the times at which each region begins and ends. In one embodiment, the region identification module 320 determines the region boundaries by searching for an expected feature in an expected range. For example, the region determination module 320 may determine a start or end boundary of a region by searching for one or more of the following in the expected range: a first peak start or valley, a last peak start or valley, a peak start or valley with the lowest magnitude, a first valley or peak end, a last valley or peak end, a valley or peak end with the lowest magnitude, a first peak start or valley or peak end, a last peak start or valley or peak end, a peak start or valley or peak end with the lowest magnitude, or a last peak end. For example, the region identification module 320 determines the retention time that is the boundary between region 1 (e.g., region 1 610) and region 2 (e.g., region 2 620) as the local minima in the retention time range corresponding to where the peak F and the peak LA1c elute, such that all of peak F would be comprised within region 1 and all of peak LA1c would be comprised within region 2, if both are present. If the expected peak feature is found in the expected range, it is used as the corresponding region boundary. If not, a default value (e.g., an absolute or normalized time) may be used for the boundary. This may account for unusual cases where expected peaks do not appear but unusual ones do. Because the region identification module 320 determines regions based on selected features, the regions may each be of a differing size (i.e., length of retention times).

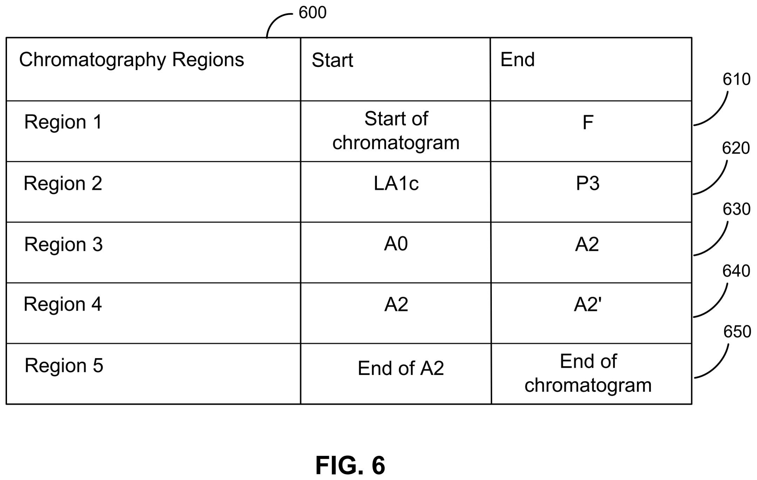

FIG. 6 is a table 600 illustrating an example division of a chromatogram into regions, according to one embodiment. The table 600 divides the chromatogram into five regions and, for each region, enumerates a start feature and an end feature. In the example illustrated by FIG. 6, region 1 610 begins at the start of the chromatogram (i.e., retention time 0.0) and ends at the retention time corresponding with the end of peak F, if F is present. That is, region 1 610 comprises a retention time range in which peaks A1a, A1b, and F elute if present. Region 2 620 begins at a retention time corresponding to the start of the peak LA1c, if present, and ends with a retention time corresponding to the end of the peak P3, if present, such that it comprises the retention time range in which peaks LA1c, HbAlc and P3 elute if present. Region 3 630, region 4 640, and region 5 650 are similarly defined by their corresponding start and stop features or times. The table 600 describes the boundaries of the regions 610, 620, 630, 640, 650 in terms of windowed components often seen in a chromatogram. However, in some embodiments, the boundaries are not dependent on the identification of these windowed components.

Referring back to FIG. 3, the template store 325 stores one or more sets of templates, or the parameters required to generate the templates as needed. Each template corresponds to an archetypical shape of a region of a chromatogram where each archetypical shape is a representation of a particular archetype of the chromatogram region. The archetypical shapes may be constructed, real, or a combination of real and constructed, where each archetypical shape mimics one or more of the peaks and troughs that are found in the particular region of the chromatogram. The real archetypical shapes come from real data sets of chromatography data, either individual or combined chromatograms. The constructed archetypical shapes are created artificially to represent an archetype, for example, by an expert constructing the expected curve. Each region is associated with a set of templates, each template having different archetypical shape. For example, the templates for region 1 may comprise archetypical shapes of the normal A1a, A1b, and F peaks, wherein each template has a different height, width, symmetry, of one or more of the peaks, and in some templates, some of the peaks may be missing entirely. In others, the expected archetypical shape of an abnormal response may be included. Each set of templates associated with a region in the template store 325 may be indexed and searchable by a variety of factors, such as height of particular peaks, absence of particular peaks, or subsets of templates known to be represent chromatogram data that is associated with certain medical conditions.

The template matching module 330 compares sets of templates to individual regions of the chromatogram to determine a template that is a best-fit match for each region. The template matching module compares a region of the chromatogram to the templates in a set of templates associated with that region stored in the template store 325. In one embodiment, the template matching module 330 slides a first template from the template store 325 across the data of the region. The template matching module 330 determines a position of the first template over the region that has the best-fit between the first template and the data of the region. The template matching module 330 may determine the best-fit position of the first template over the data of the region by determining correlation coefficient R-values between the first template and the data at different alignments of the first template and the data. Alignment may occur in one or two dimensions. For example, the alignment may include an offset in one dimension.

In some embodiments, the alignment is parameterized by a jitter range, which may be calibrated for different features based on expected retention times. The alignment of the first template and the data with the highest correlation coefficient R-value is the best-fit position of the first template and becomes the R-value associated with the first template for the data. The template matching module repeats the method of determining a best-fit correlation coefficient R-value for other templates in the template store 325. The template of a set of templates determined to have the overall highest R-value is determined by the template matching module 330 to be the best-fit match for the region. The template matching module 330 finds a best-fit match for each of the regions in the chromatogram. In other embodiments, other metrics indicating closeness of fit may be used.

In some embodiments, the template matching module 330 matches every template in a set stored in the template store 325 associated with the particular region. In other embodiments, the template matching module 330 uses determined closeness of fit metrics to expedite a determination of a best-fit match. For example, the template matching module 330 determines a correlation coefficient R-value above a threshold for a first template, which triggers additional comparison with a subset of templates that are similar to the first template. Similarly, the template matching module 330 determines a correlation coefficient R-value below a threshold for a second template, which triggers the template matching module 330 to skip comparison with a subset of templates that are similar to the second template.

The template matching module 330 may also add one or more comments. For example, the comments might identify potential diagnosis pitfalls related to the preliminary pattern, suggest further testing that would help reach a diagnosis, or identify other factors that should be considered (e.g., the ethnicity of the subject).

The result evaluation module 340 receives the output from the template matching module 330 for each region and creates a report. The result evaluation module 340 incorporates the best-fit matches for each region in an overall analysis. Calibrated percent area or other pre-processing results may be combined with the region match information to make a determination of possible medical conditions. In some embodiments, the result evaluation module 340 generates an overall best-fit template, combining each best-match template end-to-end. In another embodiment, normalized regions are overlaid individually with the best matching templates and displayed side by side as in Figure 7. The report generated by the result evaluation module 340 identifies one or more possible medical conditions or, otherwise, a determination of normalcy, or returns a result of no assignment - possible variant. For example, a region may have a best-fit match template that is associated with a likely indication of a medical condition and a recommendation for additional testing. In some embodiments, one template may indicate a plurality of possible medical conditions. In another example, a combination of the determined best-fit match templates for two or more regions with pre-processed results may indicate a medical condition, or possibly indicate a higher likelihood of the condition than either template match alone.

The report generated by the result evaluation module 340 can also include comments or advice regarding interpretation of the report, based on data associated with the individual templates of combinations of templates. For example, the comments may include an indication of the likelihood the individual has each of the one or more medical conditions, recommendations for further testing, common pitfalls associated with the one or more medical conditions, or additional information about each medical condition. For example, when testing for Beta Thalassemia, the generated report may include an HbAlc and/or A2/E result as well as information about the hemoglobin pattern, along with associated comments and notes. The added comments may alert the laboratory scientist and help the clinician in the interpretation of the result. In another example, the comments added by the result evaluation module 340 may include comments regarding features of the test results, such as the presence of a specific hemoglobin variant, or set a flag indicating that the test results should be suppressed or repeated (e.g., if the analysis suggests the results are unreliable).

FIG. 7A shows example visual representations of regions of chromatogram data overlaid with best-fit match templates. FIG. 7A includes a visual representation 710 of region 1, a visual representation 720 of region 2, a visual representation 730 of region 3, a visual representation 740 of region 4, and a visual representation 750 of region 5. The region identification module 320 divides chromatogram data 760 (see FIG. 7B) into regions, which are represented by the visual representations 710, 720, 730, 740, 750. Each visual representation of 710, 720, 730, 740, 750 of each region includes a plot of chromatogram data 712, 722, 732, 742, 752 of the region and a best-fit template 714, 724, 734, 744, 754 determined by the template matching module 330, respectively. Each visual representation 710, 720, 730, 740, 750 also includes a summary of results 716, 726, 736, 746, 756, which provide an offset value and an R value for the match between the plot of chromatogram data 712, 722, 732, 742, 752 and the best-fit template 714, 724, 734, 744, 754, for each respective region, as determined by the template matching module 330.

For example, the visual representation 710 of region 1 includes the plot of chromatogram data 712 of region 1 overlaid with a best-fit template 714 for the chromatogram data 712. The plot of chromatogram data 712 is similar in shape although not exactly the same as the best-fit template 714. For example, the peaks are shaped similarly but are slightly different heights. The template matching module 330 determined the best-fit template 714 to be an archetypical shape with the highest correlation coefficient R-value of all templates for region 1 in the template store 325 for the plot of chromatogram data 712. The correlation coefficient R-value for the best-fit template 714 and the plot of chromatogram data 712 is 0.9324, as indicated by the results 716. Other templates for region 1 in the template store 325 have lower correlation coefficient R-values than 0.9324 for the plot of chromatogram data 712. The best-fit template 714 may be associated with one or more medical conditions.

FIG. 7B shows an example of chromatogram data 760 and a report 770 that might be generated by the chromatogram analysis tool 240, according to one embodiment. The chromatogram data 760 can be divided into regions of the plots of chromatogram data 712, 722, 732, 742, 752. The chromatogram data 760 is displayed along with an overlay indicating each region number associated with the plots of chromatogram data 712, 722, 732, 742, 752. The chromatogram analysis tool 240 produces the report 770 from the example chromatogram analysis data 760. The best-fit templates 714, 724, 734, 744, 754 for the plots of chromatogram data 712, 722, 732, 742, 752 of FIG. 7A are visualizations of the best-fit match templates determined by the chromatogram analysis tool for each region of the chromatogram data 760. The report 770 is generated based on the best-fit matches 714, 724, 734, 744, 754 for each region.

The report 770 provides information about the chromatogram data 760. In the embodiment shown in FIG. 7B, the report 770 includes various information including patient information 771, a list of regions 772 with each associated best-fit match template name 773 and associated correlation coefficient R-value 774, a comment 775, and optional notes 776. In other embodiments, the report 770 may include additional or alternate information about the chromatogram data 760.

The patient information 771 includes relevant information about a patient, such as a patient ID and a rack and position indicating the location of a sample tested to produce the chromatogram data 760. In other embodiments, the patient information 771 may additionally or alternatively include a name, a blood type, responsible physician, demographic data, the date and time of the test, and other health information associated with the patient. The patient information may be stored in local storage 260 rather than being produced by the chromatogram analysis tool 240.

The list of regions 772 enumerates the regions of the chromatogram data 760. Each region in the list of regions 772 is associated with an enumerated best-fit match template name 773 and associated correlation coefficient R-value 774 for the best-fit match template. The best-fit match template names 773 enumerated in the report 770 are specific names associated with the best-fit matches 714, 724, 734, 744, 754 for each region shown in FIG. 7A. For example, the best fit match 714 is called "BARTS and H1." The correlation coefficient R-values 774 enumerated in the report 770 are the same as the R-values enumerated in the summary of results 716, 726, 736, 746, 756 of FIG. 7A. For example, the correlation coefficient R-value for region 1 is 0.934 as indicated in both FIGS. 7A and 7B.

The comment 775 indicates one or more possible medical conditions, or other medical information. The comment 76 in FIG. 7B indicates the patient likely has "BARTS with Constant Spring." In other embodiments, the comment 775 can indicate other possible medical conditions, such as the examples given below in relation to FIG. 8, or an indication of normalcy. In one embodiment, any comments 775 generated by the chromatogram analysis tool 240 are presented to a laboratory supervisor (e.g., at a terminal 130) and are only included on the report 770 if the laboratory supervisor approves them.

The notes 776 indicate a %A2 result along with an expected %A2 range for the medical disorder. The notes 776 may be produced by the pre-processing module 310 of the chromatogram analysis tool 240. In alternate embodiments, the notes 776 may include additional or alternate information.

FIG. 8 shows an example of a report 800 of a plurality of results generated by the chromatogram analysis tool 240, according to one embodiment. The report 800 includes the plurality of results obtained from a plurality of analyzed samples. The summary 800 may be displayed on a terminal 130. In some embodiments, the summary report information may be exported in a format usable by a spreadsheet application, or may be printed. The report 800 includes, for each sample, a sample name, a text name and correlation coefficient for each region, a comment, and optional notes.

The text name for each region in each sample indicates a name for a best-fit match template associated with the particular region for the particular sample, as determined by the chromatogram analysis tool 240. A column title for the text names of each region is abbreviated as "Region 1 Text" in the report 800 for region 1 and likewise for other regions. For example, sample 5 has a region 3 text name of "A0 Predominate."

The correlation coefficient for each region in each sample is a value indicating the closeness between chromatogram data within the region and the best-fit match template (e.g., R-value). A column title for the correlation coefficient is abbreviated as "Region 1 CC" in the report 800 and likewise for other regions. Each correlation coefficient is associated with the best-fit match template associated with the text name directly to the left of the respective correlation coefficient. For example, sample 5 has a region 3 correlation coefficient of 0.9246 for the best-fit match template called A0 Predominate.

The comments for each sample provide an indication of one or more possible medical conditions. The comments are provided by the chromatogram analysis tool 240. A column title for the comments is "Comments" in the report 800. As enumerated in FIG. 8, possible medical conditions indicated by the comments include but are not limited to: HbH, BARTS, Constant Spring, High F, Beta-thal major, Beta 0/E, SC, O-Arab, CC, SS, EE, Beta-thal trait.

The notes for each sample provide additional information about the sample. The comments may be provided by the chromatogram analysis tool 240 or another module. A column title for the note is "Notes" in the report 800. For example, in FIG. 8, to the first row includes a note that the percentage of the A2 peak /E peak is 0.70. The text names, correlation coefficients, comments, and notes associated with samples in the report 800 facilitate the reading of many samples at once. The report 800 may be provided in addition to or as an alternate to the report 770 of FIG. 7B.

One embodiment of the invention was run on a set of 97 chromatograms with variant responses, and results from the invention compared against manually examined assignments. Twenty-two chromatograms were of variant samples that were not contained in the template library. Twenty of the twenty-two chromatograms returned result "No assignment - possible variant" while two chromatograms returned result BARTS. Both results would trigger increased testing and scrutiny of the sample. Two additional chromatograms were from a transfused sample, which also returned result "No assignment - possible variant". Of the remaining 73 chromatograms, 67 were assigned in alignment with manual chromatogram assignment. Four of the six differences appear attributable to differing interpretations of the %A2 result, so different %A2 cutoffs for normal were used between this method and manual scrutiny. These four chromatograms were identified as normal while beta thal trait was manually assigned. The remaining two discrepancies returned a result of A2+A2' while the manual assignment was again beta thal. This result should trigger further investigation.

### COMPUTING SYSTEM ARCHITECTURE

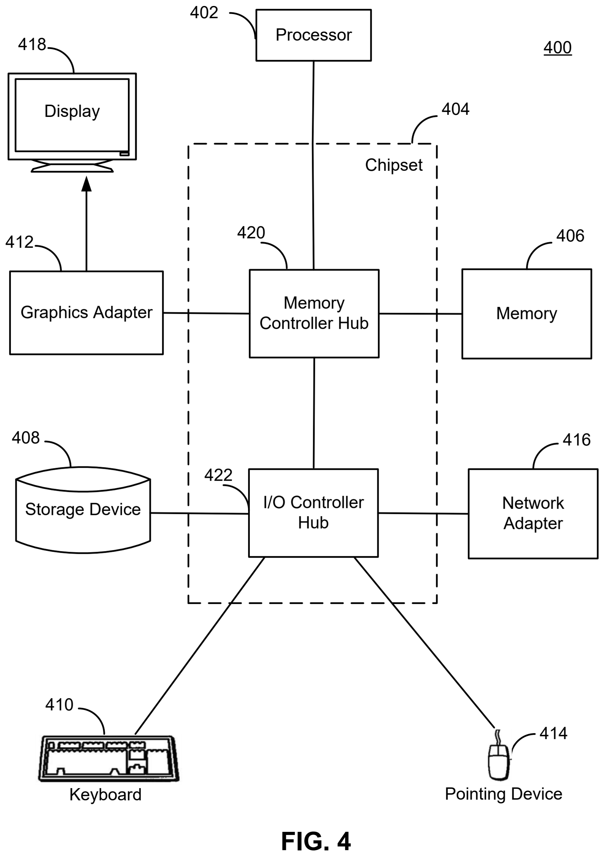

FIG. 4 illustrates an example computer 400 suitable for use as a laboratory terminal 120 or LIS 110, according to one embodiment. The example computer 400 includes at least one processor 402 coupled to a chipset 404. The chipset 404 includes a memory controller hub 420 and an input/output (I/O) controller hub 422. A memory 406 and a graphics adapter 412 are coupled to the memory controller hub 420, and a display 418 is coupled to the graphics adapter 412. A storage device 408, keyboard 410, pointing device 414, and network adapter 416 are coupled to the I/O controller hub 422. Other embodiments of the computer 400 have different architectures.

In the embodiment shown in FIG. 4, the storage device 408 is a non-transitory computer-readable storage medium such as a hard drive, compact disk read-only memory (CD-ROM), DVD, or a solid-state memory device. The memory 406 holds instructions and data used by the processor 402. The pointing device 414 is a mouse, track ball, touch-screen, or other type of pointing device, and is used in combination with the keyboard 410 (which may be an on-screen keyboard) to input data into the computer system 400. The graphics adapter 412 displays images and other information on the display 418. The network adapter 416 couples the computer system 400 to one or more computer networks.

The types of computers used by the entities of FIGS. 1 through 3 can vary depending upon the embodiment and the processing power required by the entity. For example, an LIS 110 might include a distributed database system comprising multiple blade servers working together to provide the functionality described. Furthermore, the computers can lack some of the components described above, such as keyboards 410, graphics adapters 412, and displays 418.

### EXAMPLE METHODS

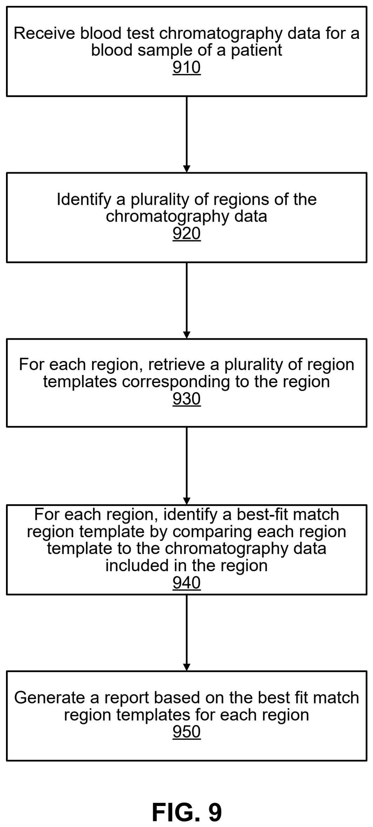

FIG. 9 is a flow-chart illustrating a method for generating a report for blood chromatography data, according to one embodiment. The steps of FIG. 9 are illustrated from the perspective of the chromatogram analysis tool 240 performing the method. However, some or all of the steps may be performed by other entities or components. In addition, some embodiments may perform the steps in parallel, perform the steps in different orders, or perform different steps.

In the embodiment shown in FIG. 9, the method begins with the chromatogram analysis tool 240 receiving 910 blood test chromatography data for a blood sample of a patient. In some embodiments, the chromatogram analysis tool 240 receives the blood test chromatography data from the laboratory equipment 120. In other embodiments, the chromatography analysis tool 240 receives the blood test chromatography data from the LIS 110. The received blood test chromatography data includes several peaks. Each peak corresponds to type of hemoglobin and has a value indicating an amount of the corresponding type of hemoglobin present in the blood sample. After receiving 910 the blood test chromatography data, the pre-processing module 310 may analyze the quality of the blood test chromatography data, perform a sample analysis, calculate special sums, or perform a baseline subtraction.

The chromatogram analysis tool 240 identifies 920 a plurality of regions of the chromatography data. The identifying 920 is performed by the region identification module 320, described in relation to FIG. 3. The regions each include chromatography data from a different range of chromatography peaks. The start and end points of each region may be identified 920 based on features of the chromatography data, or by absolute times.

The chromatography analysis tool 240, for each region, retrieves 930 a plurality of region templates corresponding to the region and identifies 940 a best-fit match region template by comparing region templates to the chromatography data included in the region. The plurality of region templates is retrieved 930 by the template matching module 330 from the template store 325. The identifying 940 of a best-fit match region is done by the template matching module 330.

The chromatography analysis tool 240 generates 950 a report based on the best-fit match region templates for each region. In one embodiment, the chromatograph analysis tool 240 generates 950 the repot additionally based on information from the pre-processing module 310, such as %A2/E. The generating 950 is performed by the result evaluation module 340. The generated 950 report may include one or more medical conditions, comments regarding the chromatogram data, and comments regarding the one or more medical conditions, including common pitfall, recommendations for additional testing, or additional information. The report may be generated 950 for display, for example, by the display subsystem, or any other display terminal.

### ADDITIONAL CONSIDERATIONS

Some portions of above description describe the embodiments in terms of algorithmic processes or operations. These algorithmic descriptions and representations are commonly used by those skilled in the data processing arts to convey the substance of their work effectively to others skilled in the art. These operations, while described functionally, computationally, or logically, are understood to be implemented by computer programs comprising instructions for execution by a processor or equivalent electrical circuits, microcode, or the like. Furthermore, it has also proven convenient at times, to refer to these arrangements of functional operations as modules, without loss of generality.

As used herein, any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Some embodiments may be described using the expression "coupled" and "connected" along with their derivatives. It should be understood that these terms are not intended as synonyms for each other. For example, some embodiments may be described using the term "connected" to indicate that two or more elements are in direct physical or electrical contact with each other. In another example, some embodiments may be described using the term "coupled" to indicate that two or more elements are in direct physical or electrical contact. The term "coupled," however, may also mean that two or more elements are not in direct contact with each other, but yet still co-operate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments. This is done merely for convenience and to give a general sense of the disclosure. This description should be read to include one or at least one and the singular also includes the plural unless it is obvious that it is meant otherwise.

Upon reading this disclosure, those of skill in the art will appreciate still additional alternative structural and functional designs for a system and a process for providing a chromatogram analysis tool that aids in hemoglobinopathy evaluation. Thus, while particular embodiments and applications have been illustrated and described, it is to be understood that the described subject matter is not limited to the precise construction and components disclosed herein and that various modifications, changes and variations which will be apparent to those skilled in the art may be made in the arrangement, operation and details of the method and apparatus disclosed. The scope of protection should be limited only by the following claims.

This application is a divisional application of European patent application number 20902851.3 (the "parent application"), also published under number EP4065975. The original claims of the parent application are repeated below as clauses in the present specification and form part of the content of this divisional application as filed.

### CLAUSES

Patentable subject matter includes but is not limited to the following:
1. A method for generating a report from blood test data, the method comprising:
   receiving blood test chromatography data for a blood sample of a patient, the data including a plurality of peaks, each peak corresponding to one or more types of hemoglobin and having a value indicating an amount of the corresponding type of hemoglobin present in the blood sample;
   identifying a plurality of regions of the chromatography data, each region including chromatography data from a different range of retention times;
   for each region:
      retrieving a plurality of region templates corresponding to the region; and
      identifying a best-fit match region template by comparing each region template to the chromatography data included in the region; and
   generating a report based on the best-fit match region templates for each region, the report indicating one or more medical conditions.
2. The method of clause 1, wherein the report includes at least one comment providing advice regarding interpretation of the report.
3. The method of clause 2, wherein the advice regarding interpretation of the report includes at least one of: a common pitfall associated with the one or more medical conditions, a recommendation for additional testing, or additional information about the medical condition.
4. The method of clause 1, further comprising:
   providing the report for display at a terminal.
5. The method of clause 1, wherein each regions of the plurality of regions have corresponding predetermined ranges, wherein each range is defined by a start feature and an end feature in the chromatogram.
6. The method of clause 1, wherein at least two of the predetermined ranges are of different lengths.
7. The method of clause 1, further comprising:
   determining an indication of quality of the chromatography data; and
   generating a notification if the indication of the quality is below a predetermined threshold.
8. The method of clause 1, wherein for each region, identifying the best-fit match region template comprises,
   for each region template:
      determining a maximum correlation coefficient R-value between the chromatography data of the region and the region template;
   determining the best-fit match region template based on the maximum correlation coefficient R-values.
9. The method of clause 8, wherein determining the maximum correlation coefficient R-value for a given region template comprises:
   sliding the given region template across the region of the chromatography data;
   calculating R-values for different positions of the given region template within the region of the chromatography data; and
   selecting a largest one of the R-values as the maximum R-value for the given region template.
10. The method of clause 1, wherein each template represents an archetypical shape of a chromatogram within the region, including one or more peaks.
11. A non-transitory computer-readable medium storing computer program instructions executable by a processor to perform operations comprising:
   receiving blood test chromatography data for a blood sample of a patient, the data including a plurality of peaks, each peak corresponding to one or more types of hemoglobin indicating an amount of the corresponding one or more types of hemoglobin present in the blood sample;
   identifying a plurality of regions of the chromatography data, each region including chromatography data from a different range of retention times;
   for each region:
      retrieving a plurality of region templates corresponding to the region; and
      identifying a best-fit match region template by comparing each region template to the chromatography data included in the region; and
   generating a report based on the best-fit match region templates for each region, the report indicating one or more medical conditions.
12. The non-transitory computer-readable medium of clause 11, wherein the report includes at least one comment providing advice regarding interpretation of the report.
13. The non-transitory computer-readable medium of clause 12, wherein the advice regarding interpretation of the report includes at least one of: a common pitfall associated with the one or more medical conditions, a recommendation for additional testing, or additional information about the medical condition.
14. The non-transitory computer-readable medium of clause 11, storing instructions further comprising:
   providing the report for display at a terminal.
15. The non-transitory computer-readable medium of clause 11, wherein each regions of the plurality of regions have corresponding predetermined ranges, wherein each range is defined by a start feature and an end feature in the chromatogram.
16. The non-transitory computer-readable medium of clause 11, wherein at least two of the predetermined ranges are of different lengths.
17. The non-transitory computer-readable medium of clause 11, further comprising:
   determining an indication of quality of the chromatography data to determine; and
   generating a notification if the indication of the quality is below a predetermined threshold.
18. The non-transitory computer-readable medium of clause 11, wherein for each region, identifying the best-fit match region template further comprises,
   for each region template:
      determining a R-value between the chromatography data of the region the region template;
   determining the best-fit match region based on the highest R-value.
19. The non-transitory computer-readable medium of clause 11, wherein each template represents hemoglobin composition of an archetypical shape of a chromatogram within the region, including one or more peaks.
20. A chromatography device for generating a report from blood test data, the chromatography device comprising:
   a port for injecting a blood sample extracted from a patient;
   one or more processors; and
   a computer readable medium storing computer program code that, when executed, causes the one or more processors to perform operations including:
      receive blood test chromatography data for a blood sample of a patient, the data including a plurality of peaks, each peak corresponding to one or more types of hemoglobin and having a value indicating an amount of the corresponding type of hemoglobin present in the blood sample;
      identify a plurality of regions of the chromatography data, each region including chromatography data from a different range of retention times;
      for each region:
         retrieve a plurality of region templates corresponding to the region; and
         identify a best-fit match region template by comparing each region template to the chromatography data included in the region; and
      generate a report based on the best-fit match region templates for each region.

## Claims

1. A laboratory system comprising:
a laboratory device that generates blood test data from a blood sample of a patient, the blood test data including a plurality of peaks, each peak corresponding to one or more types of hemoglobin and having a value indicating an amount of the corresponding type of hemoglobin present in the blood sample; and
a laboratory terminal configured to:
provide the blood test data to an analysis tool;
receive, from the analysis tool, a report, wherein the report indicates a best-fit match region template for each of a plurality of regions of the blood test data, each region including blood test data from a different range of retention times; and
display the report.

2. The laboratory system of claim 1, wherein the report includes at least one comment providing advice regarding interpretation of the report.

3. The laboratory system of claim 2, wherein the advice regarding interpretation of the report includes at least one of: a common pitfall associated with one or more medical conditions, a recommendation for additional testing, or additional information about the medical condition.

4. The laboratory system of claim 1, wherein the analysis tool is software executing on the laboratory terminal.

5. The laboratory system of claim 1, wherein each of the regions of the plurality of regions have corresponding predetermined ranges, wherein each range is defined by a start feature and an end feature in the blood test data, and wherein, optionally, at least two of the corresponding predetermined ranges are of different lengths.

6. The laboratory system of claim 1, wherein the analysis tool is further configured to:
determine an indication of quality of the blood test data; and
send a notification to the laboratory terminal if the indication of the quality is below a predetermined threshold.

7. The laboratory system of claim 1, wherein the laboratory device is a chromatography system that comprises a port for injecting a blood sample extracted from a patient.

8. The laboratory system of claim 1, wherein the best-fit match region template for a region is determined using correlation coefficient R-values for a plurality of possible region templates, and wherein, optionally, each of the plurality of possible region templates represents an archetypical shape of a chromatogram within the region, including one or more peaks.

9. One or more non-transitory computer-readable media storing computer program instructions that, when executed, cause one or more computing devices to perform operations comprising:
generating blood test data from a blood sample of a patient, the blood test data including a plurality of peaks, each peak corresponding to one or more types of hemoglobin and having a value indicating an amount of the corresponding type of hemoglobin present in the blood sample;
providing the blood test data to an analysis tool;
receiving, from the analysis tool, a report, wherein the report indicates a best-fit match region template for each of a plurality of regions of the blood test data, each region including blood test data from a different range of retention times; and displaying the report.

10. The one or more non-transitory computer-readable media of claim 9, wherein the report includes at least one comment providing advice regarding interpretation of the report, wherein, optionally, the advice regarding interpretation of the report includes at least one of:
a common pitfall associated with one or more medical conditions, a recommendation for additional testing, or additional information about the medical condition.

11. The one or more non-transitory computer-readable media of claim 9, wherein the analysis tool is software executing on a laboratory terminal, and wherein, optionally, each of the regions of the plurality of regions have corresponding predetermined ranges, wherein each range is defined by a start feature and an end feature in the blood test data.

12. The one or more non-transitory computer-readable media of claim 11, wherein at least two of the corresponding predetermined ranges are of different lengths.

13. The one or more non-transitory computer-readable media of claim 9, wherein the analysis tool is configured to:
determine an indication of quality of the blood test data; and
send a notification to the laboratory terminal if the indication of the quality is below a predetermined threshold.

14. The one or more non-transitory computer-readable media of claim 9, wherein the blood test data is generated by a chromatography system that comprises a port for injecting a blood sample extracted from a patient.

15. The one or more non-transitory computer-readable media of claim 9, wherein the best-fit match region template for a region is determined using correlation coefficient R-values for a plurality of possible region templates, wherein, optionally, each of the plurality of possible region templates represents an archetypical shape of a chromatogram within the region, including one or more peaks.
